Europäisches Patentamt

(19) European Patent Office

Office européen des brevets

(11) Publication number : **0 475 894 A1**

## (12) EUROPEAN PATENT APPLICATION

(21) Application number : **91810665.9**

(22) Date of filing : **21.08.91**

(51) Int. Cl.⁵ : **A61K 31/19, A61K 9/22**

(30) Priority : **27.08.90 US 572886**

(43) Date of publication of application :
**18.03.92 Bulletin 92/12**

(84) Designated Contracting States :
**AT BE CH DE DK ES FR GB GR IT LI LU NL SE**

(71) Applicant : **WARNER-LAMBERT COMPANY**
**201 Tabor Road**
**Morris Plains New Jersey 07950 (US)**

(72) Inventor : **Ghebre-Sellassie, Isaac**
**359 Dell Place**
**Stanhope, New Jersey 07874 (US)**
Inventor : **Fawzi, Mahdi B.**
**11 Timberline Drive**
**Flanders, New Jersey 07836 (US)**

(74) Representative : **Silbiger, Jakob, Dr.**
**c/o CAPSUGEL AG Fabrikmattenweg 2-4**
**CH-4144 Arlesheim (CH)**

(54) **Gemfibrozil formulations.**

(57)    Improved oral formulations are prepared by granulating a blend of gemfibrozil, an alkali metal or alkaline earth metal salt, and an effective amount of at least one moisture scavenger with minimal water, and drying the resultant particulate.

EP 0 475 894 A1

Jouve, 18, rue Saint-Denis, 75001 PARIS

The present invention relates to improved formulations of gemfibrozil.

Gemfibrozil, or 5-(2,5-dimethylphenoxy)-2,2-dimethylpentanoic acid, is a widely used antihyperlipoproteinemic agent. Physically the chemical is a crystalline material which melts in the range of 61° to 63°C (hexane) and exhibits a boiling point of 158-159°$_{0.02}$C. The substance is non-hygroscopic and generally compatible with common pharmaceutical excipients but has very poor solubility in water. This is particularly true in a highly acidic medium (such as is encountered in the stomach) since its apparent pKa is 4.7.

The typical daily dose is high, generally about 1200 mg, probably because of the poor water solubility. This dosage generally is administered using, for example, two capsules of 300 mg or a single compressed tablet of 600 mg, administration in each case being b.i.d.

The present invention pertains to improvements in gemfibrozil formulations which improve the compound's dissolution profile and thus increase the drug's blood levels upon oral administration.

U.S. Patent No. 4,716,033 discloses a medicament adsorbate such as magnesium aluminum silicate having a medicament, including inter alia gemfibrozil, and surfactant adsorbed thereon.

U.S. Patent No. 4,753,800 discloses a medicament adsorbate having a medicament, including inter alia gemfibrozil, dispersed in an edible wax adsorbed thereon.

U.S. Patent No. 4,814,354 discloses pharmaceutical compositions of an anion exchange resin lipid regulator, such as cholestyramine or cholestipol, and gemfibrozil.

U.S. Patent No. 4,778,676 discloses a chewable confection delivery system of coated cholestyramine and a confectionery matrix.

U.S. Patent No. 4,816,264 discloses an oral delivery system having a core portion of drug and a cellulosic gelling polymer and a semipermeable membrane around the core.

U.S. Patent No. 4,865,850 discloses a method of expelling fat from the gastrointestinal tract by administering non-biodegradable collagen particles having fat receptors, including inter alia gemfibrozil, on their surface.

EP-A 295,637 A2 discloses pharmaceutical compositions in which a lipid regulating component, including inter alia gemfibrozil, is combined with inhibitor of acylCoA:cholesterol acyltransferase.

EP-A 261,693 A1 discloses a lipid regulating component, including inter alia gemfibrozil, which has been pre-treated to render it stable until such time as it reaches the proximal section of the intestines.

PCT WO 88/05296 discloses pharmaceutical compositions in which a lipid regulating component, including inter alia gemfibrozil, is combined with inhibitor of 3-hydroxy-3-methylglutaryl coenzyme A reductase.

According to the present invention there is provided an oral formulation of gemfibrozil comprising a dried particulate prepared from an aqueous granulation of gemfibrozil, and an alkali metal or alkaline earth metal salt of a weak acid sufficient to produce a pH of at least 5.

The present invention is based on the discovery when gemfibrozil is granulated with a pharmaceutically acceptable buffering salt of a strong base and weak acid, and optionally an effective amount of at least one moisture scavenger, the resultant oral formulations exhibit improved dissolution rates.

More specifically, the present invention relates to an improved oral formulation of gemfibrozil comprising a dried buffered particulate prepared from an aqueous granulation of gemfibrozil, and an alkali metal or alkaline earth metal salt of a weak acid such as a carbonate, bicarbonate, acetate, citrate, phosphate, or the like, and optionally with an effective amount of moisture scavenger.

Preferably the alkali metal or alkaline earth metal carbonate, bicarbonate, or phosphate is the sodium salt such as sodium carbonate, sodium bicarbonate, sodium acetate, sodium citrate, or sodium phosphate. Sodium carbonate is particularly preferred.

The amount of buffering salt added will depend on its relative basicity. Generally the amount will be at least sufficient to produce a pKa (upon dissolution of the granulation in water) of 5.0 or more. Additional quantities up to the stoichiometric amount can be used and in some instances use of full stoichiometric amounts is desirable. Amounts greater than the stoichiometric amount offer little advantage.

According to the invention, a blend of gemfibrozil and the buffering salt, optionally with a moisture scavenger, is granulated with minimal water, optionally with buffered solution or a surfactant.

The moisture scavenger can be, for example, a hydratable polysaccharide such as microcrystalline cellulose, hydroxypropyl cellulose, a soluble disaccharide such as lactose, and the like or a silica such as silica gel. Suitable pharmaceutically acceptable surfactants include polysorbates, pluronics, alkali metal salts of fatty alcohol sulfates, such as sodium lauryl sulfate, salts of fatty acids such as sodium oleate and triethanolamine oleate, and the like.

Following granulation, the resultant particulate is thoroughly dried and then is ready for combination with conventional pharmaceutical excipients used in the formulation of tablets and capsules, as for example talc, lactose, starch, lubricants such as calcium or magnesium stearate, additional silicon dioxide, and the like.

The following is a typical formulation:

| | |
|---|---|
| Gemfibrozil | 600.00 |
| Sodium Carbonate | 127.02 |
| Silica Gel | 30.00 |
| Sodium Lauryl Sulfate | 6.00 |
| Water | 330.00 |
| Hydroxypropyl cellulose | 24.00 |

The gemfibrozil, sodium carbonate, and silica gel are mixed in a Hobart bowl. To this mixture then is slowly added with mixing a solution of the sodium lauryl sulfate in about 54 g of water, or a buffered solution having a pH above about 6.5, such as sodium phosphate dibasic followed by a solution of the hydroxypropyl cellulose in the remaining water. After about 30 minutes of mixing, the granulation end point is reached and the granulation then is dried at 40°C for 24 hours.

The foregoing particulate can be directly introduced into capsules, Alternatively, the particulate can be milled and combined with excipients for formulation of compressed tablets or pellets. For example, the dried particulate is milled through a #2AA screen at medium speed, blended with 120 g of lactose, 10 g of talc, and 12 g of magnesium stearate, and compressed into tablet cores.

Capsules prepared from the particulate of the present invention were tested to evaluate their rate of release dissolution in pH 7.5 phosphate buffer.

The results are as follows :

### Percent Released

| Time (minutes) | Commercial Product | Above Formulation |
|---|---|---|
| 10 | 53.7 | 85.5 |
| 20 | 75.0 | 97.5 |
| 30 | 83.5 | 99.1 |
| 40 | 87.9 | 99.6 |
| 50 | 90.8 | 100.0 |
| 60 | 92.9 | 100.0 |

As can be seen from the above, the formulation according to the present invention demonstrates a significant increase in the rate of dissolution, over 99% being released in 30 minutes.

Pellets and tablets can be further coated with water-soluble polymers, enter-soluble polymers, or water-insoluble polymers to produce, respectively, immediate, enteric or sustained release products.

The present invention is summarised by the following clauses, numbered 1 to 20 and is defined by the claims appended thereafter.

1. An improved oral formulation of gemfibrozil comprising a dried particulate prepared from an aqueous granulation of gemfibrozil, and an alkali metal or alkaline earth metal salt of a weak acid sufficient to produce a pH of at least 5.

2. An improved oral formulation of gemfibrozil according to 1 including an effective amount of at least one moisture scavenger.

3. An improved formulation of gemfibrozil according to 1 wherein the alkali metal or alkaline earth metal salt is a carbonate, bicarbonate, acetate, citrate or phosphate.

4. An improved oral formulation of gemfibrozil according to 1 wherein the alkali metal or alkaline earth metal salt is sodium bicarbonate.

5. An improved oral formulation of gemfibrozil according to 1 wherein the alkali metal or alkaline earth metal is sodium carbonate.

6. An improved oral formulation of gemfibrozil according to 1 wherein the alkali metal or alkaline earth metal is sodium phosphate.

7. An improved oral formulation of gemfibrozil according to 2 wherein the moisture scavenger includes a polysaccharide.

8. An improved oral formulation of gemfibrozil according to 7 wherein the polysaccharide is microcrystalline cellulose.

9. An improved oral formulation of gemfibrozil according to 7 wherein the polysaccharide is hydroxypropyl cellulose.

10. An improved oral formulation of gemfibrozil according to 2 wherein the moisture scavenger includes a silica.

11. In the process for the preparation of oral formulations of gemfibrozil, the steps which comprise granulating a blend of gemfibrozil and an alkali metal or alkaline earth metal salt of a weak acid with minimal water, and drying the resultant granulated particulate.

12. The process according to 11 wherein the blend contains an effective amount of at least one moisture scavenger.

13. The process according to 11 wherein the alkali metal or alkaline earth metal salt is a carbonate, bicarbonate, acetate, citrate or phosphate.

14. The process according to 11 wherein the alkali metal or alkaline earth metal salt is sodium bicarbonate.

15. The process according to 11 wherein the alkali metal or alkaline earth metal salt is sodium phosphate.

16. The process according to 11 wherein the alkali metal or alkaline earth metal salt is sodium carbonate.

17. The process according to 12 wherein the moisture scavenger includes a polysaccharide.

18. The process according to 17 wherein the polysaccharide is microcrystalline cellulose.

19. The process according to 17 wherein the polysaccharide is hydroxypropyl cellulose.

20. The process according to 12 wherein the moisture scavenger includes a silica.

## Claims

1. An oral formulation of gemfibrozil comprising a dried particulate prepared from an aqueous granulation of gemfibrozil, and an alkali metal or alkaline earth metal salt of a weak acid sufficient to produce a pH of at least 5.

2. An oral formulation of gemfibrozil according to claim 1, including an effective amount of at least one moisture scavenger.

3. An oral formulation of gemfibrozil according to either of claims 1 or 2, wherein the alkali metal or alkaline earth metal salt is a carbonate, bicarbonate, acetate, citrate or phosphate.

4. An oral formulation of gemfibrozil according to any preceding claim, wherein the alkali metal or alkaline earth metal salt is sodium bicarbonate or sodium carbonate.

5. An oral formulation of gemfibrozil according to any one of claims 1 to 3, wherein the alkali metal or alkaline earth metal salt is sodium phosphate.

6. An oral formulation of gemfibrozil according to any one of claims 2 to 5, wherein the moisture scavenger includes a polysaccharide.

7. An oral formulation of gemfibrozil according to any one of claims 2 to 6, wherein the polysaccharide is microcrystalline cellulose, and preferably is hydroxypropyl cellulose.

8. An oral formulation of gemfibrozil according to claim 2 wherein the moisture scavenger includes a silica.

9. A process for the preparation of oral formulations of gemfibrozil according to any preceding claim, comprising the steps of granulating a blend of gemfibrozil and an alkali metal or alkaline earth metal salt of a weak acid with minimal water, and drying the resultant granulated particulate.

10. The use of the oral formulation of gemfibrozil of any one of claims 1 to 8, in the manufacture of a medicament for the treatment of hyperlipoproteinemia.

**European Patent Office**

## EUROPEAN SEARCH REPORT

Application Number

| DOCUMENTS CONSIDERED TO BE RELEVANT | | | EP 91810665.9 |
|---|---|---|---|

| Category | Citation of document with indication, where appropriate, of relevant passages | Relevant to claim | CLASSIFICATION OF THE APPLICATION (Int. Cl.5) |
|---|---|---|---|
| A | EP - A - 0 381 219 (WARNER-LAMBERT COMPANY) * Claims; page 1, lines 6-9 * -- | 1,2,6, 7,10 | A 61 K 31/19 A 61 K 9/22 |
| A | EP - A - 0 381 218 (WARNER-LAMBERT COMPANY) * Abstract; page 1, lines 6-9 * -- | 1,10 | |
| A | EP - A - 0 381 220 (WARNER-LAMBERT COMPANY) * Abstract; page 1, lines 6-10 * ---- | 1,10 | |
| | | | TECHNICAL FIELDS SEARCHED (Int. Cl.5) A 61 K |

The present search report has been drawn up for all claims

| Place of search | Date of completion of the search | Examiner |
|---|---|---|
| VIENNA | 04-12-1991 | WOLF |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
............................................................
& : member of the same patent family, corresponding
 document

EPO FORM 1503 03.82 (P0401)